(19) Europäisches Patentamt
European Patent Office
Office européen des brevets

(11) Publication number: 0 230 127 A1

# (12) EUROPEAN PATENT APPLICATION

(21) Application number: 86309739.0

(22) Date of filing: 15.12.86

(51) Int. Cl.4: C 07 D 277/28

(30) Priority: 18.12.85 US 810136

(43) Date of publication of application: 29.07.87 Bulletin 87/31

(84) Designated Contracting States: AT BE CH DE ES FR GB GR IT LI LU NL SE

(71) Applicant: ELI LILLY AND COMPANY, Lilly Corporate Center, Indianapolis Indiana 46285 (US)

(72) Inventor: Jackson, Billy Grinnell, 4230 Lincoln Road, Indianapolis Indiana 46208 (US)
Inventor: Slomski, Bruce Andrew, 3209 East 10th Street Apartment 16-1, Bloomington Indiana 47401 (US)

(74) Representative: Tapping, Kenneth George et al, Lilly Industries Limited Patent Department Erl Wood Manor, Windlesham Surrey, GU20 6PH (GB)

## (54) Synthesis of nizatidine.

(57) The present invention provides a process for preparing nizatidine comprising reacting 4-[[(2-aminoethyl)thio]methyl]-N,N-dimethyl-2-thiazolemethanamine with a 1-alkoxy-N-methyl-2-nitroetheneamine.

EP 0 230 127 A1

SYNTHESIS OF NIZATIDINE

Nizatidine is the generic name given to the compound N-[2-[[[2-[(dimethylamino)methyl]-4-thiazolyl]-methyl]thio]ethyl]-N'-methyl-2-nitro-1,1-ethenediamine, an anti-ulcer compound taught by Pioch in U.S. Patent No. 4,375,547. The patent discloses the synthesis of nizatidine by the reaction of 4-[[(2-aminoethyl)thio]-methyl]-N,N-dimethyl-2-thiazolemethanamine with 1,1-dimethylthio-2-nitroethene to provide the corresponding 2-nitro-1-methylthio-1-etheneamine derivative, which is subsequently converted to nizatidine upon reaction with monomethylamine. The process disclosed in the patent produces methanethiol gas as a by-product, which is known to produce an unpleasant odor that requires the use of extra purification equipment to eliminate.

The present invention provides a process for preparing nizatidine comprising reacting a 1-alkoxy-N-methyl-2-nitroetheneamine with 4-[[(2-aminoethyl)thio]-methyl]-N,N-dimethyl-2-thiazolemethanamine in water. The present process does not produce methanethiol gas, and is therefore an especially valuable process for preparing nizatidine on a large scale wherein cost is an important consideration.

The present invention provides a process for preparing nizatidine having the formula

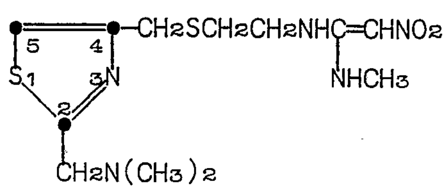

comprising reacting a 1-alkoxy-N-methyl-2-nitroetheneamine of the formula

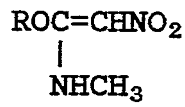

wherein R is $C_{1-4}$ alkyl, with 4-[[(2-aminoethyl)thio]methyl]-N,N-dimethyl-2-thiazolemethanamine having the formula

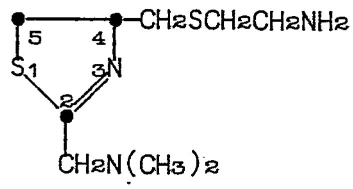

The present process can be carried out by combining a 1-alkoxy-N-methyl-2-nitroetheneamine with 4-[[(2-aminoethyl)thio]methyl]-N,N-dimethyl-2-thiazolemethanamine in a polar solvent such as water in a suitable reaction vessel. Preferably, the thiazolemethanamine is added to a suspension of the nitroetheneamine in water.

Water is preferably employed as the solvent in the present process. The amount of water used in the present process is not critical, but no more than necessary to suspend the starting thiazolemethanamine and nitroetheneamine need be used.

The amount of 1-alkoxy-N-methyl-2-nitroetheneamine employed in the present process will be in the range of about one to about two molar equivalents for each molar equivalent of thiazolemethanamine employed.

Preferably, the two starting reactants are employed on about an equimolar basis.

The present process is substantially complete after about 10 minutes to about 24 hours when conducted at a temperature in the range of about 10°C to about 100°C, preferably after about 30 minutes to about four hours when conducted at a temperature in the range of about 15°C to about 60°C.

R is preferably methyl or ethyl.

The product of the present process may be isolated by standard procedures. Typically, the solution is neutralized with a suitable base, such as sodium bicarbonate, and the mixture is extracted with a water immiscible organic solvent such as methylene chloride. The organic phase is isolated and the solvent is then evaporated, typically under vacuum. The residue is finally purified, if desired, by standard techniques such as crystallization from common solvents or purification over solid supports such as silica gel or alumina.

The process of the present invention has been found to provide the product consistently in high purity, so that the compound may be used without additional expensive purification steps. The present process has an added advantage in industrial scale synthesis in that the process does not produce methanethiol gas as a by-product, a compound which is known to produce an unpleasant odor. The elimination of this stench removes the need for special equipment and handling processes, as well as suitable means of disposal as would be otherwise necessary when this gas is produced.

The starting materials employed in the present process are known in the art and readily prepared by prior art processes. The thiazolemethanamine is disclosed in U.S. Patent No. 4,375,547. The 1-alkoxy-N-methyl-2-nitroetheneamines are preferably synthesized by reacting a trialkyloxonium tetrafluoroborate with N-methyl-2-nitroacetamide in an aprotic solvent.

The following Examples further illustrate the process of the present invention. The Examples are not intended to be limiting to the scope of the invention in any respect and should not be so construed.

## Example 1

### Synthesis of Nizatidine

#### A. 1-Methoxy-N-methyl-2-nitroetheneamine

A solution of 3.33 g (0.028 mol) of N-methyl-2-nitroacetamide and 4.8 g (0.032 mol) of trimethyloxonium tetrafluoroborate in 100 ml of methylene chloride was stirred at about 25°C for about 20 hours. To the mixture was added 100 ml of a cold saturated sodium bicarbonate solution. The layers were separated and the aqueous phase was extracted with 30 ml methylene chloride. The organic extracts were combined and dried over anhydrous magnesium sulfate. The mixture was filtered and the volatile constituents were evaporated under vacuum to provide 1.30 g of 1-methoxy-N-methyl-2-nitroetheneamine as a yellow solid. Yield 35.2%. The structure of the product was verified by proton NMR.

B. To a mixture of 0.66 g (0.005 mol) of 1-methoxy-N-methyl-2-nitroetheneamine suspended in 5 ml of water at room temperature was added 1.17 g (0.005 mol) of 4-[[(2-aminoethyl)thio]methyl]-N,N-dimethyl-2-thiazolemethanamine. A slight exotherm occurred as the solid components went into solution. The reaction mixture was stirred at room temperature for 2½ hours and 10 ml of a saturated sodium bicarbonate solution was added. The mixture was extracted twice with 10 ml portions of methylene chloride. The organic extracts were combined and dried over anhydrous magnesium sulfate and the organic solution was concentrated under vacuum to provide an oil. Ethanol was added to the oil, and the resulting solution was concentrated to an oil. The oil was solubilized in ethanol and ethyl acetate, and seed crystals of nizatidine were added to the solution. The solution was cooled, and the precipitated solid was collected by vacuum filtration to provide 1.31 g of nizatidine. A thin layer chromatograph of the solid indicated the presence of minor impurities. The solid was recrystallized from ethanol to provide 0.65 g of nizatidine which was substantially pure as determined by thin layer chromatography. Yield 39.3%.

## Example 2

### Synthesis of Nizatidine

A mixture of 8.48 g (0.035 mol) of 95% pure 4-[[(2-aminoethyl)thio]methyl]-N,N-dimethyl-2-thiazolemethanamine, 8.03 g (0.056 mol) of 1-ethoxy-N-methyl-2-nitroetheneamine and 200 ml of water was heated at about 55°C with stirring for about four hours. The progress of the reaction was followed by HPLC. The temperature of the reaction mixture was raised to 65°C and maintained for an additional 90 minutes, and then to 75°C for an additional 60 minutes. One gram of potassium carbonate was added to the reaction mixture, and the resulting mixture was heated at about 50°C for about 15 minutes. HPLC analysis indicated the presence of 88.8 mole percent of nizatidine in the mixture. The reaction mixture was cooled, diluted by the addition of 100 ml of methylene chloride, and 40 g sodium chloride was added. The organic phase was separated, and the aqueous phase was extracted with 100 ml of methylene chloride and then 50 ml of methylene chloride. The organic phases were combined and dried over anhydrous magnesium sulfate. The volume of the organic phase was reduced to about 100 ml by evaporation under vacuum. The methylene chloride solution was combined with 100 ml of ethanol, and the resulting solution was concentrated under vacuum to dryness. To the residue was added 90 ml of ethyl acetate and the resulting mixture was stirred at room temperature for about 20 minutes. The mixture was allowed to stand overnight at room temperature. The precipitated solid was collected by vacuum filtration and washed with 50 ml of fresh ethyl acetate to provide 6.25 g of nizatidine following vacuum drying. The purity of the product was 95% by HPLC. Yield 53.9%.

Claims

1. A process for preparing nizatidine having the formula

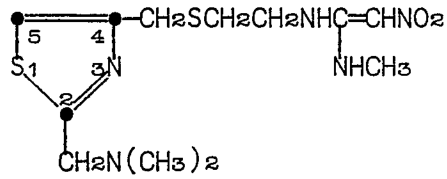

,

comprising reacting a 1-alkoxy-N-methyl-2-nitroethene-amine of the formula

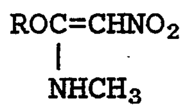

wherein R is $C_{1-4}$ alkyl, with 4-[[(2-aminoethyl)thio]-methyl]-N,N-dimethyl-2-thiazolemethanamine having the formula

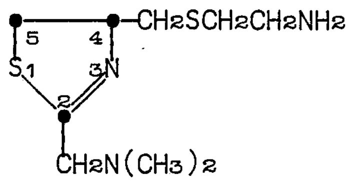

2. A process according to Claim 1 wherein R is methyl or ethyl and the reaction is carried out in water.

European Patent Office

# EUROPEAN SEARCH REPORT

0230127

Application number

EP 86 30 9739

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 200 578 (ALGIERI-CRENSHAW) * Columns 7,8 * --- | 1 | C 07 D 277/28 |
| A | EP-A-0 116 452 (ELI LILLY) * Pages 1,2 * --- | 1 | |
| D,A | EP-A-0 049 618 (ELI LILLY) * Page 13 * ----- | 1 | |
| | | | TECHNICAL FIELDS SEARCHED (Int. Cl.4) |
| | | | C 07 D 277/00 |

The present search report has been drawn up for all claims

| Place of search | Date of completion of the search | Examiner |
|---|---|---|
| THE HAGUE | 26-03-1987 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document